# EUROPÄISCHE PATENTSCHRIFT

(11) **EP 4 175 902 B1**
(45) Veröffentlichungstag und Bekanntmachung des Hinweises auf die Patenterteilung: **26.03.2025**
(21) Anmeldenummer: 21737368.7
(22) Anmeldetag: 24.06.2021
(51) Int. Cl.: B65G 47/08, B65G 47/84

(54) **VERFAHREN UND VORRICHTUNG ZUM HANDHABEN VON (HYGIENE-)PRODUKTEN**
METHOD AND DEVICE FOR HANDLING (HYGIENE) PRODUCTS
PROCÉDÉ ET DISPOSITIF DE MANIPULATION DE PRODUITS (D'HYGIÈNE)

(30) Priorität: 01.07.2020 DE 102020117315
(43) Veröffentlichungstag der Anmeldung: 10.05.2023
(73) Patentinhaber: Focke & Co. (GmbH & Co. KG), 27283 Verden (DE)
(72) Erfinder: VOCKS, Matthias, 26683 Strücklingen (DE); PRAHM, Andreas, 26676 Barßel (DE); WILHELM, Arthur, 26670 Uplengen (DE); KRAMER, Torsten, 26125 Oldenburg (DE)
(74) Vertreter: Ellberg, Nils
(86) Internationale Anmeldenummer: PCT/EP2021/067300
(87) Internationale Veröffentlichungsnummer: WO 2022/002743

(56) Entgegenhaltungen:
- EP-A1- 0 709 315
- WO-A2-2008/135201
- CN-A- 108 438 327
- DE-A1- 102012 208 029
- DE-A1- 3 835 058
- US-A- 6 006 491
- US-A1- 2007 289 842

## Beschreibung

Die Erfindung betrifft ein Verfahren zum Handhaben von (Hygiene-)Produkten, insbesondere von Windeln, wobei die Produkte mit einem Zuförderer in einer ersten Transportrichtung in Fächer eines Abförderers, insbesondere eines Gruppierförderers, transportiert werden, der die Produkte in einer zweiten Transportrichtung transportiert, wobei die zweite Transportrichtung quer, insbesondere senkrecht, zur ersten Transportrichtung verläuft, und wobei zwischen dem Zuförderer und dem Abförderer wenigstens ein Zwischenförderer angeordnet ist, der zur Verringerung der Transportgeschwindigkeit der Produkte in der ersten Transportrichtung dient, gemäß dem Oberbegriff des Anspruchs 1.

Die Erfindung betrifft weiterhin eine entsprechende Vorrichtung gemäß dem Oberbegriff des Anspruchs 8.

Bei der Herstellung von Sammelverpackungen für (Hygiene-)Produkte wird oft so vorgegangen, dass die Produkte einem Fächerförderer zugeführt werden. Vom Fächerförderer werden die Produkte dann in Gruppen einer entsprechenden Verpackungsmaschine zugeführt und in eine gemeinsame Umhüllung eingeschlagen. Bei aus der Praxis bekannten Fächerförderern werden die Produkte direkt in die Fächer eingebracht. Der Transportweg der Produkte wird dabei durch einen Anschlag gebremst, gegen den die in den Fächerförderer eingeförderten Produkte stoßen.

In der EP 0 709 315 A1, die als nächstliegender Stand der Technik angesehen wird, ist eine Vorrichtung gezeigt, welche eine Geschwindigkeitsreduktionseinheit verwendet, um zugeführte Produkte zu verlangsamen und in einen ersten Fächer-Förderer zu überführen. Dabei ist die Geschwindigkeitsreduktionseinheit als weiterer, relativ zum ersten Fächer-Förderer geneigter Fächer-Förderer ausgebildet. EP 0 709 315 A1 offenbart die Merkmale der Oberbegriffe der Ansprüche 1 und 8.

Ein Nachteil dieser Vorgehensweise wird darin gesehen, dass die Produkte bei der Übergabe von der Geschwindigkeitsreduktionseinheit zum Fächer-Förderer ihre Orientierung ändern. Dadurch haben die Produkte ein erhöhtes Risiko gekippt relativ zu den Fächern des Fächerförderers positioniert zu werden und im weiteren Produktionsablauf Probleme zu verursachen.

Hiervon ausgehend liegt der Erfindung die Aufgabe zugrunde, Verfahren und Vorrichtungen der eingangs genannten Art weiterzuentwickeln, insbesondere im Hinblick auf die Beseitigung der Nachteile des Standes der Technik.

Ein Verfahren zur Lösung dieser Aufgabe weist die Merkmale des Anspruchs 1 auf. Es ist demnach vorgesehen, dass aus den Fächern herausragende Bereiche der Produkte durch wenigstens einen weiteren Fächerförderer stabilisiert werden, insbesondere durch Anlage der Produkte an Trennwänden des weiteren Fächerförderers, wobei der oder jeder weitere Fächerförderer kontinuierlich in der zweiten Transportrichtung angetrieben wird und vorzugsweise oberhalb des Zwischenförderers und/oder des Abförderers angeordnet ist.

In einer bevorzugten Ausführungsform der Erfindung kann vorgesehen sein, dass der Zwischenförderer wenigstens im Wesentlichen parallel zur zweiten Transportrichtung verläuft.

Die Verzögerung kann dadurch erreicht werden, dass die Transportgeschwindigkeit der Produkte in der ersten Transportrichtung durch temporäre Anlage der Produkte am Zwischenförderer bzw. Organen desselben verringert wird.

Mit anderen Worten kann vorgesehen sein, dass die Transportgeschwindigkeit der Produkte in der ersten Transportrichtung durch Reibung zwischen den Produkten und dem Zwischenförderer bzw. Organen desselben verringert wird.

Vorzugsweise ist vorgesehen, dass Zwischenförderer und Abförderer kontinuierlich und mit gleicher Geschwindigkeit in der zweiten Transportrichtung angetrieben werden, und dass beide Förderer als Fächerförderer ausgebildet sind und Trennwände zur Trennung von Fächern des jeweiligen Förderers aufweisen, wobei die beiden Förderer derart zueinander ausgerichtet angetrieben werden, dass die jeweiligen Fächer und Trennwände wenigstens teilweise fluchtend angeordnet sind, sodass die Produkte nacheinander durch den Zuförderer durch ein Fach des Zwischenförderers hindurch in ein Fach des Abförderers transportiert werden, wobei die Produkte durch teilweise Anlage bzw. Reibung an den Trennwänden des Zwischenförderers und des Abförderers hinsichtlich ihrer Transportgeschwindigkeit in der ersten Transportrichtung abgebremst werden.

Vorzugsweise kann in der erfindungsgemäßen Konstellation auch vorgesehen sein, dass der oder die weiteren Fächerförderer schräg zur Transportebene des Zwischenförderers und/oder Abförderers verlaufen, sodass die Trennwände des oder der weiteren Fächerförderer während des Transports der Produkte in der zweiten Transportrichtung allmählich von den Produkten abgezogen werden.

Eine weitere Besonderheit kann darin bestehen, dass zwischen dem Zwischenförderer und dem Abförderer eine Weiche angeordnet ist, mit der die Produkte während des Transports in der zweiten Transportrichtung vorzugsweise vollständig auf den Abförderer geschoben werden und/oder auf dem Abförderer ausgerichtet werden.

Eine Vorrichtung zur Lösung der eingangs genannten Aufgabe weist die Merkmale des Anspruchs 8 auf. Es ist demnach entsprechend vorgesehen, dass aus den Fächern herausragende Bereiche der Produkte durch wenigstens einen weiteren Fächerförderer stabilisiert werden, insbesondere durch Anlage der Produkte an Trennwänden des weiteren Fächerförderers, wobei der oder jede weitere Fächerförderer kontinuierlich in der zweiten Transportrichtung angetrieben ist und vorzugsweise oberhalb des Zwischenförderers und/oder des Abförderers angeordnet ist.

Vorzugsweise ist vorgesehen, dass die Vorrichtung dazu eingerichtet ist, den Zwischenförderer und Abförderer kontinuierlich und mit gleicher Geschwindigkeit in der zweiten Transportrichtung anzutreiben, und dass beide Förderer als Fächerförderer ausgebildet sind und Trennwände zur Trennung von Fächern des jeweiligen Förderers aufweisen, wobei die Vorrichtung dazu eingerichtet ist, die beiden Förderer derart zueinander ausgerichtet anzutreiben, dass die jeweiligen Fächer und Trennwände wenigstens teilweise fluchtend angeordnet sind, sodass Produkte nacheinander durch den Zuförderer durch ein Fach des Zwischenförderers hindurch in ein Fach des Abförderers transportiert werden, wobei die Produkte durch teilweise Anlage an den Trennwänden des Zwischenförderers und des Abförderers hinsichtlich ihrer Transportgeschwindigkeit in der ersten Transportrichtung abgebremst werden.

In einer bevorzugten Ausführungsform der Erfindung kann vorgesehen sein, dass die Fächer derart bemessen sind, dass diese nur geringfügig breiter als das jeweilige Produkt sind, sodass das Produkt durch das Fach bzw. dessen Trennwände quer zur ersten Transportrichtung abgestützt wird.

In der erfindungsgemäßen Konstellation kann ferner vorgesehen sein, dass der oder die weiteren Fächerförderer schräg zur Transportebene des Zwischenförderers und/oder Abförderers verlaufen, sodass die Trennwände des oder der weiteren Fächerförderer während des Transports der Produkte in der zweiten Transportrichtung allmählich von den Produkten abgezogen werden, insbesondere bevor die Produkte in einen Umlenkbereich des weiteren Fächerförderers gelangen.

Bevorzugt ist vorgesehen, dass zwischen dem Zwischenförderer und dem Abförderer eine Weiche angeordnet ist, zum vorzugsweise vollständigen Abschub der Produkte während des Transports in der zweiten Transportrichtung auf den Abförderer und/oder zur Ausrichtung der Produkte auf dem Abförderer.

Eine weitere Besonderheit kann darin bestehen, dass der Zwischenförderer in der zweiten Transportrichtung mit kürzerer Erstreckung als der Abförderer ausgebildet ist und sich vorzugsweise nur in einem Übergabebereich für die Produkte erstreckt.

Ein bevorzugtes Detail der Erfindung kann darin bestehen, dass mehrere Trennwände zu Fächersegmenten mit mehreren voneinander beabstandeten Trennwänden zusammengefasst sind.

Weiterhin kann vorgesehen sein, dass die Fächersegmente über ein Schnellverschlusssystem mit dem entsprechenden Förderer gekoppelt sind, insbesondere mit einem Zahnriemen des jeweiligen Förderers.

In einer bevorzugten Ausführungsform der Erfindung kann vorgesehen sein, dass oberhalb des Zwischenförderers und oberhalb des Abförderers jeweils ein zusätzlicher Fächerförderer angeordnet ist, der in der zweiten Transportrichtung angetrieben ist.

Ein bevorzugtes Ausführungsbeispiel der Erfindung wird nachfolgend anhand von Zeichnungen beschrieben. In diesen zeigen:
- Fig. 1: eine Vorrichtung zum Handhaben von (Hygiene-)Produkten in schematischer räumlicher Darstellung,

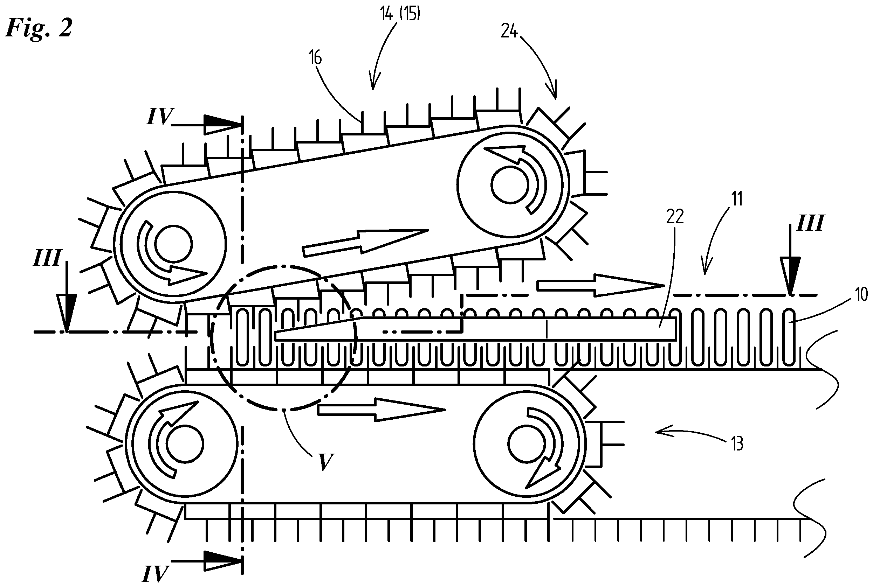

- Fig. 2: einen Vertikalschnitt durch die Vorrichtung entlang Schnittlinie II - II in Fig. 1,

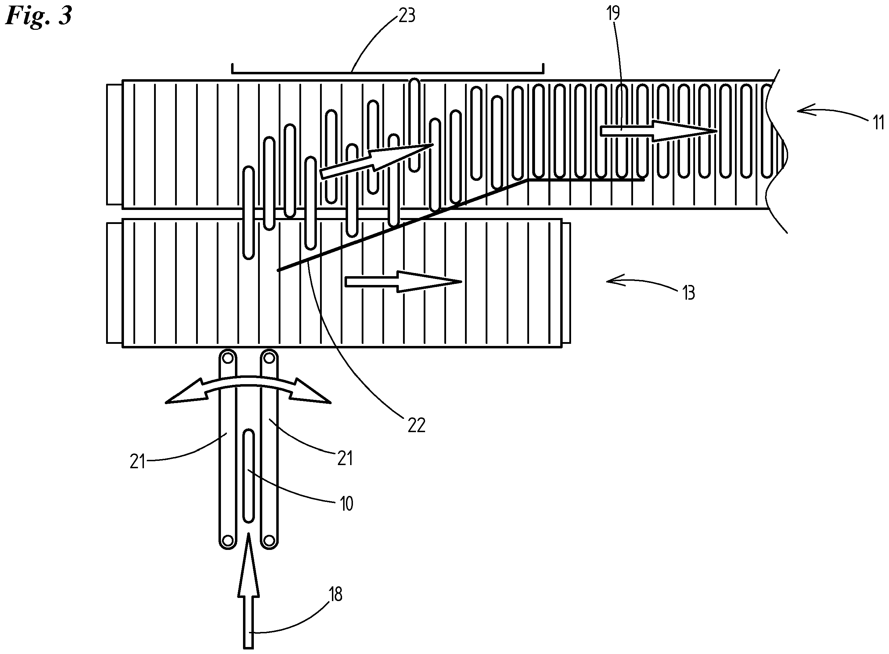

- Fig. 3: einen Horizontalschnitt durch die Vorrichtung entlang Schnittlinie III - III in Fig. 2,

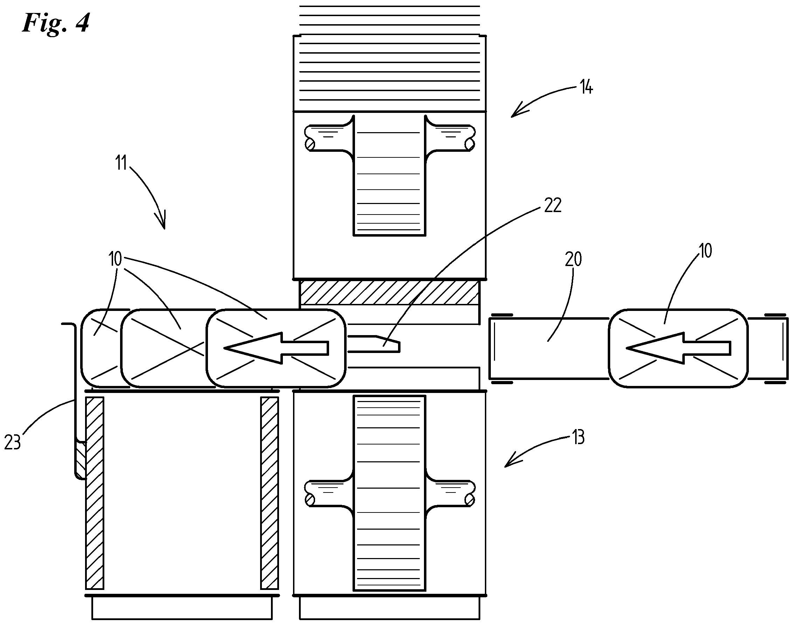

- Fig. 4: einen Vertikalschnitt durch die Vorrichtung entlang Schnittlinie IV - IV in Fig. 2, und
- Fig. 5: eine Einzelheit der Vorrichtung im Bereich V in Fig. 2.

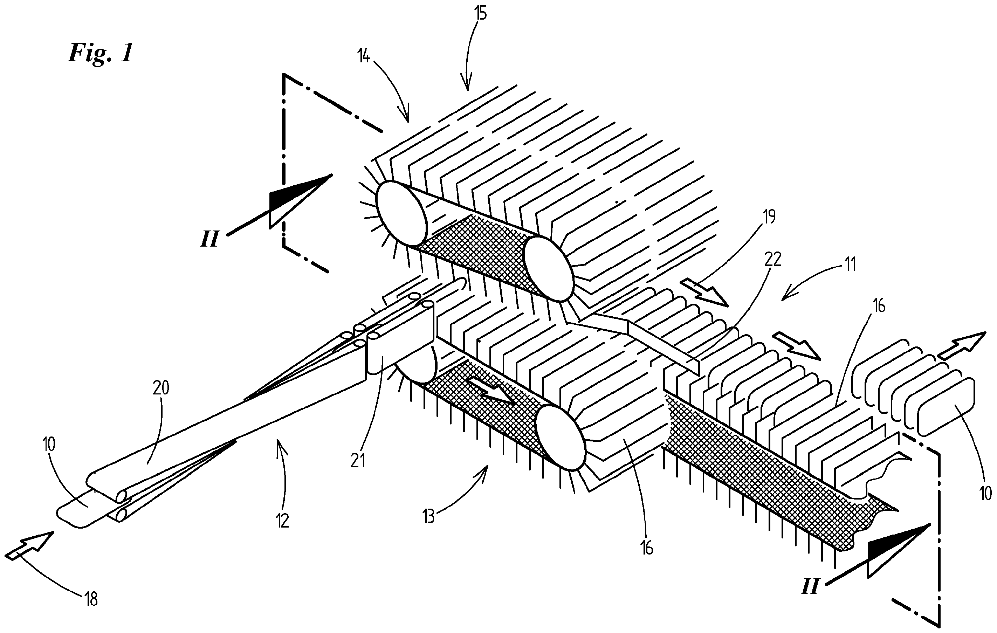

Die in Fig. 1 in wesentlichen Teilen gezeigte Vorrichtung dient zur Handhabung von Windeln. Es versteht sich, dass die Vorrichtung natürlich auch zur Handhabung von ähnlichen Hygieneprodukten geeignet ist, sodass nachfolgend allgemein von Produkten 10 besprochen wird.

Die Produkte 10 werden einem Abförderer 11 zugeführt. Hierzu dient ein Zuförderer 12. Zwischen Zuförderer 12 und Abförderer 11 ist ein Zwischenförderer 13 angeordnet. Oberhalb von Abförderer 11 und Zwischenförderer 13 befinden sich zwei weitere Fächerförderer 14, 15.

Der Antrieb der Förderer erfolgt durchgehend kontinuierlich. Entsprechend werden auch die Produkte 10 kontinuierlich bewegt.

Der Zwischenförderer 13, der Abförderer 11 und die beiden weiteren Förderer 14, 15 sind allesamt als Fächerförderer ausgebildet, d.h. am Umfang des vorzugsweise endlosen Fördertrums der Förderer sind Trennwände 16 angeordnet, die vorzugsweise senkrecht zum Fördertrum stehen und die zur Bildung von Fächern 17 der Förderer dienen, in denen vorzugsweise ein Produkt 10, eventuell aber auch mehrere Produkte 10, Aufnahme finden. Es versteht sich, dass die Förderer nicht zwangsläufig als Fächerförderer ausgebildet sein müssen, sondern dass auch durch den Begriff ähnliche Konstellationen erfasst werden, bei denen Fächer gebildet sind, die zur Aufnahme der zu fördernden Produkte 10 geeignet sind.

Die Vorrichtung verfügt im Wesentlichen über zwei Transportrichtungen. Eine erste Transportrichtung 18 wird durch den Zuförderer 12 vorgegeben. Die zweite Transportrichtung 19 verläuft quer, vorzugsweise im Wesentlichen senkrecht hierzu. Die zweite Transportrichtung 19 entspricht der Längserstreckung des Zwischenförderers 13, des Abförderers 11 und der beiden Fächerförderer 14, 15.

Der Zuförderer 12 ist zweiteilig ausgebildet und verfügt zunächst über ein Paar von Twistbändern 20, sowie hieran anschließend ein Paar von Einlaufbändern 21. Die Produkte 10 werden den Twistbändern 20 flachliegend zugeführt und während des Transports mittels der Twistbänder 20 aufgerichtet. Danach werden die Produkte 10 an die Einlaufbänder 21 übergeben, welche dazu dienen, die Produkte 10 nacheinander dem Abförderer 11 zuzuführen.

Die Zuführung der Produkte 10 von den Einlaufbändern 21 an den Abförderer 11 erfolgt durch den Zwischenförderer 13 bzw. dessen Fächer 17 hindurch. Die Abgabegeschwindigkeit der Einlaufbänder 21 wird entsprechend so gewählt, dass die Produkte 10 durch die Fächer 17 des Zwischenförderers 13 hindurch in entsprechende Fächer 17 des Abförderers 11 transportiert werden. Dabei kommen die Produkte 10 zwangsläufig mit den Trennwänden 16 des Zwischenförderers 13 in Kontakt und werden durch die auftretende Reibung abgebremst.

Die Breite der Fächer 17 quer zur ersten Transportrichtung 18 wird dabei so gewählt, dass ein geeigneter Abbremseffekt eintritt. Auch die Trennwände 16 des Abförderers 11 können zum Abbremsen der Produkte 10 herangezogen werden, sodass die Produkte 10 am Ende ordnungsgemäß in den Fächern 17 des Abförderers 11 landen oder allenfalls noch mit einer geringen Geschwindigkeit gegen einen sicherheitshalber im Anschluss an den Abförderer 11 angeordneten Anschlag 23 stoßen, ohne dass eine nennenswerte Deformation der Produkte 10 erfolgt.

Da der Zwischenförderer 13 und der Abförderer 11 während des Transports der Produkte 10 kontinuierlich angetrieben werden, ist vorgesehen, dass das stromabwärts liegende Ende der Einlaufbänder 21 seitlich verschwenkt werden kann und mit den Fächern des Zwischenförderers 13 mitläuft. Auf diese Weise wird verhindert, dass die Produkte 10 gegen die Trennwände 16 des Zwischenförderers 13 stoßen.

Die Trennwände 16 des Zwischenförderers 13 und des Abförderers 11 sind zueinander ausgerichtet, sodass fluchtende Fächer 17 auf dem Zwischenförderer 13 und dem Abförderer 11 gebildet werden.

Ferner ist im Verlauf des Abförderers 11 eine Weiche 22 vorgesehen, welche die Produkte 10 gegebenenfalls auf den Abförderer 11 schiebt und/oder die dort befindlichen Produkte 10 in einer Reihe ausrichtet.

Am Ende des Abförderers 11 werden die Produkte 10 in Gruppen aus den Fächern 17 des Abförderers 11 ausgeschoben und weiter verarbeitet.

Eine weitere Besonderheit besteht hinsichtlich der Anordnung und Ausrichtung der weiteren Fächerförderer 14, 15. Im Grundriss gesehen befinden sich die weiteren Fächerförderer 14, 15 jeweils oberhalb des Zwischenförderers 13 und des Abförderers 11. Darüber hinaus sind die weiteren Fächerförderer 14, 15 gegenüber der Transportebene des Zwischenförderers 13 und des Abförderers 11 geneigt angeordnet.

Die weiteren Fächerförderer 14, 15 dienen dazu, einen oberen Rand der in den Fächern 17 stehenden Produkte 10 abzustützen und so ein Abknicken der Produkte 10 zu verhindern. Auf der anderen Seite muss dafür gesorgt werden, dass der Bereich, in dem die weiteren Fächerförderer 14, 15 zur Abstützung dienen können endet, bevor die Produkte 10 in den Umlenkungsbereich der weiteren Fächerförderer 14, 15 gelangen. Ansonsten würde nämlich dies dazu führen, dass die Produkte 10 im Umlenkbereich der weiteren Fächerförderer 14, 15 durch die Trennwände 16 des ersten bzw. zweiten Fächerförderers 14, 15 umgeknickt werden. Entsprechend sind die weiteren Fächerförderer 14,15 leicht ansteigend in Transportrichtung geneigt, sodass die Produkte 10 nicht mehr durch die Trennwände 16 der weiteren Fächerförderer 14, 15 seitlich gestützt werden, bevor der Umlenkung ich der beiden Förderer erreicht wird.

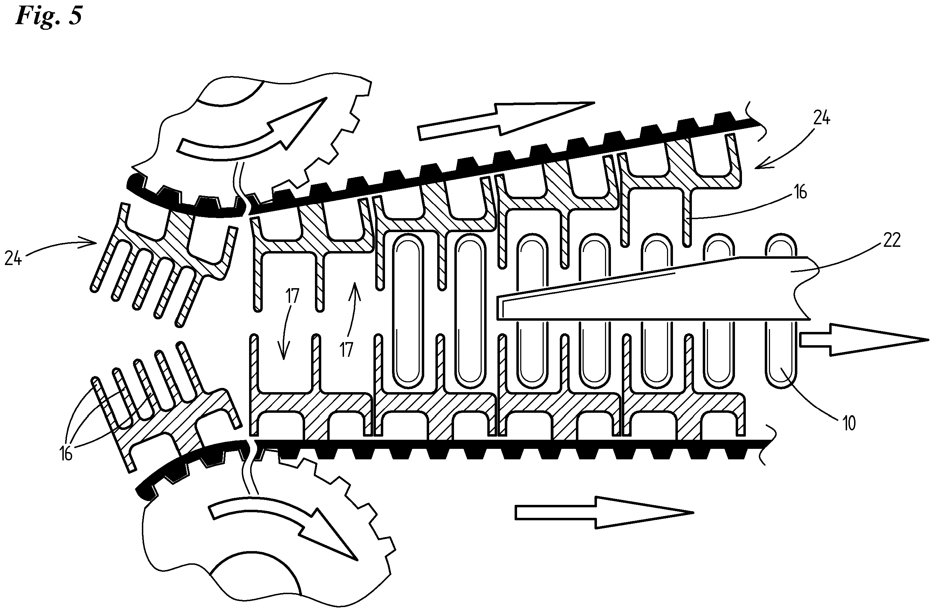

Wie Fig. 5 zeigt, sind jeweils mehrere Trennwände 16 zu einem Fächersegment 24 zusammengefasst. Gezeigt sind Fächersegmente 24 mit zwei Trennwänden 16 und beispielhaft jeweils ein Fächersegment mit fünf Trennwänden 16. Die Fächersegmente 24 sind über ein Schnellverschlusssystem mit dem Fördertrum der Förderer verbunden.

Zusammenfassend lässt sich die Arbeitsweise der Vorrichtung mit anderen Worten etwa wie folgt beschreiben:
Die Produkte 10 kommen flach liegend von einem Konverter und werden zu einem verdrillten Förderband 20 gefahren. In dem verdrillten Förderband 20 werden die Produkte 10 senkrecht gestellt und mit hoher Geschwindigkeit zu einem Stacker gefördert.

Der Stacker besteht aus vier Fächergurten 11, 13, 14, 15. Die untere Führung der Produkte 10 übernehmen die Fächersegmente 24 der beiden Fächergurte 11, 13 und die obere Führung der Produkte 10 übernehmen die Fächersegmente 24 der beiden Fächergurte 14, 15.

Nachdem die Produkte 10 in die Fächer 17 eingetaucht sind, verlieren sie an Geschwindigkeit und kommen in dem Bereich zwischen den vorderen Fächerförderern 13, 14 und hinteren Fächerförderern 11, 15 zum Stillstand. Sie haben einen besonders langen Auslaufweg und stoßen in der Regel nicht an den Anschlag 23. Dadurch ist die Vorrichtung besonders produktschonend. Das bedeutet, dass die Bremskraft lediglich durch die Reibung an der Seitenfläche des Produkts 10 aufgebracht wird und keine Deformation durch den Anschlag 23 stattfindet.

Wenn der Verzögerungsvorgang der Produkte 10 abgeschlossen ist, werden die oberen Fächerförderer 14, 15 während der Querbewegung nach oben gezogen. Sie dienen nur der beidseitigen Führung während des Einschusses, damit keine Produkte 10 in nicht zugeordneten Fächern 17 zum Stillstand kommen.

Damit die Fächersegmente 24 des vorderen und hinteren Fächerförderers 14, 15 in der Umlenkung umschwenken können, ohne gegen die obere Produktkante zu stoßen, müssen sie aus dem Produktbereich gezogen werden. Dies wird durch Schrägstellung der Fächerförderer 14, 15 bewerkstelligt.

Weil die Lage der Produkte 10 in den Fächern 17 unbestimmt ist, wenn sie zum Stillstand kommen, werden sie mit einer Weiche 22 während der Querbewegung ausgerichtet. Nach dieser Egalisierung werden sie mit dem Abförderer 11 einem Bagger zugefügt.

## Patentansprüche

1. Verfahren zum Handhaben von (Hygiene-)Produkten (10), insbesondere von Windeln, wobei die Produkte (10) mit einem Zuförderer (12) in einer ersten Transportrichtung (18) in Fächer (17) eines Abförderers (11), insbesondere eines Gruppierförderers, transportiert werden, der die Produkte (10) in einer zweiten Transportrichtung (19) transportiert, wobei die zweite Transportrichtung (19) quer, insbesondere senkrecht, zur ersten Transportrichtung (18) verläuft, und wobei zwischen dem Zuförderer (12) und dem Abförderer (11) wenigstens ein Zwischenförderer (13) angeordnet ist, der zur Verringerung der Transportgeschwindigkeit der Produkte (10) in der ersten Transportrichtung (18) dient, **dadurch gekennzeichnet, dass** aus den Fächern (17) herausragende Bereiche der Produkte (10) durch wenigstens einen weiteren Fächerförderer (14, 15) stabilisiert werden, insbesondere durch Anlage der Produkte (10) an Trennwänden (16) des weiteren Fächerförderers (14, 15), wobei der oder jeder weitere Fächerförderer (14, 15) kontinuierlich in der zweiten Transportrichtung (19) angetrieben wird und vorzugsweise oberhalb des Zwischenförderers (13) und/oder des Abförderers (11) angeordnet ist.

2. Verfahren nach Anspruch 1, **dadurch gekennzeichnet, dass** der Zwischenförderer (13) wenigstens im Wesentlichen parallel zur zweiten Transportrichtung (19) verläuft.

3. Verfahren nach Anspruch 1 oder 2, **dadurch gekennzeichnet, dass** die Transportgeschwindigkeit der Produkte (10) in der ersten Transportrichtung (18) durch temporäre Anlage der Produkte (10) am Zwischenförderer (13) bzw. Organen desselben verringert wird.

4. Verfahren nach Anspruch 1 oder einem der anderen vorhergehenden Ansprüche, **dadurch gekennzeichnet, dass** die Transportgeschwindigkeit der Produkte (10) in der ersten Transportrichtung (18) durch Reibung zwischen den Produkten (10) und dem Zwischenförderer (13) bzw. Organen desselben verringert wird.

5. Verfahren nach Anspruch 1 oder einem der anderen vorhergehenden Ansprüche, **dadurch gekennzeichnet, dass** Zwischenförderer (13) und Abförderer (11) kontinuierlich und mit gleicher Geschwindigkeit in der zweiten Transportrichtung (19) angetrieben werden, und dass beide Förderer als Fächerförderer ausgebildet sind und Trennwände (16) zur Trennung von Fächern (17) des jeweiligen Förderers aufweisen, wobei die beiden Förderer derart zueinander ausgerichtet angetrieben werden, dass die jeweiligen Fächer (17) und Trennwände (16) wenigstens teilweise fluchtend angeordnet sind, sodass die Produkte (10) nacheinander durch den Zuförderer (12) durch ein Fach (17) des Zwischenförderers (13) hindurch in ein Fach (17) des Abförderers (11) transportiert werden, wobei die Produkte (10) durch teilweise Anlage bzw. Reibung an den Trennwänden (16) des Zwischenförderers (13) und des Abförderers (11) hinsichtlich ihrer Transportgeschwindigkeit in der ersten Transportrichtung (18) abgebremst werden.

6. Verfahren nach Anspruch 1 oder einem der anderen vorhergehenden Ansprüche, **dadurch gekennzeichnet, dass** der oder die weiteren Fächerförderer (14, 15) schräg zur Transportebene des Zwischenförderers (13) und/oder Abförderers (11) verlaufen, sodass die Trennwände (16) des oder der weiteren Fächerförderer (14, 15) während des Transports der Produkte (10) in der zweiten Transportrichtung (19) allmählich von den Produkten (10) abgezogen werden.

7. Verfahren nach Anspruch 1 oder einem der anderen vorhergehenden Ansprüche, **dadurch gekennzeichnet, dass** zwischen dem Zwischenförderer (13) und dem Abförderer (11) eine Weiche (22) angeordnet sind, mit der die Produkte (10) während des Transports in der zweiten Transportrichtung (19) vorzugsweise vollständig auf den Abförderer (11) geschoben werden und/oder auf dem Abförderer (11) ausgerichtet werden.

8. Vorrichtung zum Handhaben von (Hygiene-)Produkten (10), insbesondere von Windeln, mit einem Zuförderer (12) zum Transport der Produkte (10) in einer ersten Transportrichtung (18) in Fächer (17) eines Abförderers (11), insbesondere eines Gruppierförderers, wobei der Abförderer (11) zum Transport der Produkte (10) in einer zweiten Transportrichtung (19) eingerichtet ist, und wobei die zweite Transportrichtung (19) quer, insbesondere senkrecht, zur ersten Transportrichtung (18) verläuft, und wobei zwischen dem Zuförderer (12) und dem Abförderer (11) wenigstens ein Zwischenförderer (13) angeordnet ist, zur Verringerung der Transportgeschwindigkeit der Produkte (10) in der ersten Transportrichtung (18), **dadurch gekennzeichnet, dass** aus den Fächern (17) herausragende Bereiche der Produkte (10) durch wenigstens einen weiteren Fächerförderer (14, 15) stabilisiert werden, insbesondere durch Anlage der Produkte (10) an Trennwänden (16) des weiteren Fächerförderers (14, 15), wobei der oder jede weitere Fächerförderer (14, 15) kontinuierlich in der zweiten Transportrichtung (19) angetrieben ist und vorzugsweise oberhalb des Zwischenförderers (13) und/oder des Abförderers (11) angeordnet ist.

9. Vorrichtung nach Anspruch 8, **dadurch gekennzeichnet, dass** die Vorrichtung dazu eingerichtet ist, den Zwischenförderer (13) und Abförderer (11) kontinuierlich und mit gleicher Geschwindigkeit in der zweiten Transportrichtung (19) anzutreiben, und dass beide Förderer als Fächerförderer ausgebildet sind und Trennwände (16) zur Trennung von Fächern (17) des jeweiligen Förderers aufweisen, wobei die Vorrichtung dazu eingerichtet ist, die beiden Förderer derart zueinander ausgerichtet anzutreiben, dass die jeweiligen Fächer (17) und Trennwände (16) wenigstens teilweise fluchtend angeordnet sind, sodass Produkte (10) nacheinander durch den Zuförderer (12) durch ein Fach (17) des Zwischenförderers (13) hindurch in ein Fach (17) des Abförderers (11) transportiert werden, wobei die Produkte (10) durch teilweise Anlage an den Trennwänden (16) des Zwischenförderers (13) und des Abförderers (11) hinsichtlich ihrer Transportgeschwindigkeit in der ersten Transportrichtung (18) abgebremst werden.

10. Vorrichtung nach Anspruch 8 oder 9, **dadurch gekennzeichnet, dass** die Fächer (17) derart bemessen sind, dass diese nur geringfügig breiter als das jeweilige Produkt (10) sind, sodass das Produkt (10) durch das Fach (17) bzw. dessen Trennwände (16) quer zur ersten Transportrichtung (18) abgestützt wird.

11. Vorrichtung nach Anspruch 8 oder einem der anderen vorhergehenden Ansprüche 9 bis 11, **dadurch gekennzeichnet, dass** der oder die weiteren Fächerförderer (14, 15) schräg zur Transportebene des Zwischenförderers (13) und/oder Abförderers (11) verlaufen, sodass die Trennwände (16) des oder der weiteren Fächerförderer (14, 15) während des Transports der Produkte (10) in der zweiten Transportrichtung (19) allmählich von den Produkten (10) abgezogen werden, insbesondere bevor die Produkte (10) in einen Umlenkbereich des weiteren Fächerförderers (14, 15) gelangen.

12. Vorrichtung nach Anspruch 8 oder einem der anderen vorhergehenden Ansprüche 9 bis 11, **dadurch gekennzeichnet, dass** zwischen dem Zwischenförderer (13) und dem Abförderer (11) eine Weiche (24) angeordnet ist, zum vorzugsweise vollständigen Abschub der Produkte (10) während des Transports in der zweiten Transportrichtung (19) auf den Abförderer (11) und/oder zur Ausrichtung der Produkte (10) auf dem Abförderer (11).

13. Vorrichtung nach Anspruch 8 oder einem der anderen vorhergehenden Ansprüche 9 bis 12, **dadurch gekennzeichnet, dass** der Zwischenförderer (13) in der zweiten Transportrichtung (19) mit kürzerer Erstreckung als der Abförderer (11) ausgebildet ist und sich vorzugsweise nur in einem Übergabebereich für die Produkte (10) erstreckt.

14. Vorrichtung nach Anspruch 9 oder einem der anderen vorhergehenden Ansprüche 8 bis 13, **dadurch gekennzeichnet, dass** mehrere Trennwände (16) zu Fächersegmenten (24) mit mehreren voneinander beabstandeten Trennwänden (16) zusammengefasst sind.

15. Vorrichtung nach Anspruch 14 oder einem der anderen vorhergehenden Ansprüche 8 bis 13, **dadurch gekennzeichnet, dass** die Fächersegmente (24) über ein Schnellverschlusssystem mit dem entsprechenden Förderer gekoppelt sind, insbesondere mit einem Zahnriemen des jeweiligen Förderers.

16. Vorrichtung nach Anspruch 8 oder einem der anderen vorhergehenden Ansprüche 8 bis 14, **dadurch gekennzeichnet, dass** oberhalb des Zwischenförderers (13) und oberhalb des Abförderers (11) jeweils ein zusätzlicher Fächerförderer (14, 15) angeordnet ist, der in der zweiten Transportrichtung (19) angetrieben ist.

## Claims

1. A method for handling (hygiene) products (10), in particular diapers, the products (10) being transported by way of a feed conveyor (12) in a first transport direction (18) into compartments (17) of a discharge conveyor (11), in particular of a grouping conveyor, which transports the products (10) in a second transport direction (19), the second transport direction (19) running transversely, in particular perpendicularly, with respect to the first transport direction (18), and wherein at least one intermediate conveyor (13), which serves to decrease the transfer speed of the products (10) in the first transport direction (18), is arranged between the feed conveyor (12) and the discharge conveyor (11), **characterized in that** regions of the products (10) which protrude out of the compartments (17) are stabilized by way of at least one further compartment conveyor (14, 15), in particular by way of contact of the products (10) with dividing walls (16) of the further compartment conveyor (14, 15), the or each further compartment conveyor (14, 15) being driven continuously in the second transport direction (19) and preferably being arranged above the intermediate conveyor (13) and/or the discharge conveyor (11).

2. The method as claimed in claim 1, **characterized in that** the intermediate conveyor (13) runs at least substantially parallel to the second transport direction (19).

3. The method as claimed in claim 1 or 2, **characterized in that** the transport speed of the products (10) in the first transport direction (18) is decreased by way of temporary contact of the products (10) with the intermediate conveyor (13) or members thereof.

4. The method as claimed in claim 1 or one of the other preceding claims, **characterized in that** the transport speed of the products (10) in the first transport direction (18) is decreased by way of friction between the products (10) and the intermediate conveyor (13) or members thereof.

5. The method as claimed in claim 1 or one of the other preceding claims, **characterized in that** intermediate conveyor (13) and discharge conveyor (11) are driven continuously and at the same speed in the second transport direction (19), and **in that** the two conveyors are configured as compartment conveyors and have dividing walls (16) for dividing compartments (17) of the respective conveyor, the two conveyors being driven in a manner which is oriented with respect to one another in such a way that the respective compartments (17) and dividing walls (16) are arranged so as to be aligned at least partially, with the result that the products (10) are transported one after another by way of the feed conveyor (12) through a compartment (17) of the intermediate conveyor (13) into a compartment (17) of the discharge conveyor (11), the products (10) being braked with regard to their transport speed in the first transport direction (18) by way of partial contact or friction with the dividing walls (16) of the intermediate conveyor (13) and of the discharge conveyor (11).

6. The method as claimed in claim 1 or one of the other preceding claims, **characterized in that** the compartment conveyor or the further compartment conveyors (14, 15) run obliquely with respect to the transport plane of the intermediate conveyor (13) and/or discharge conveyor (11), with the result that the dividing walls (16) of the compartment conveyor or the further compartment conveyors (14, 15) are withdrawn gradually from the products (10) during the transport of the products (10) in the second transport direction (19).

7. The method as claimed in claim 1 or one of the other preceding claims, **characterized in that** a diverter (22) is arranged between the intermediate conveyor (13) and the discharge conveyor (11), by way of which diverter (22) the products (10) are preferably pushed completely onto the discharge conveyor (11) and/or are aligned on the discharge conveyor (11) during the transport in the second transport direction (19).

8. An apparatus for handling (hygiene) products (10), in particular diapers, with a feed conveyor (12) for transporting the products (10) in a first transport direction (18) into compartments (17) of a discharge conveyor (11), in particular of a grouping conveyor, the discharge conveyor (11) being configured to transport the products (10) in a second transport direction (19), and the second transport direction (19) running transversely, in particular perpendicularly, with respect to the first transport direction (18), and wherein at least one intermediate conveyor (13) is arranged between the feed conveyor (12) and the discharge conveyor (11), for decreasing the transport speed of the products (10) in the first transport direction (18), **characterized in that** regions of the products (10) which protrude out of the compartments (17) are stabilized by way of at least one further compartment conveyor (14, 15), in particular by way of contact of the products (10) with dividing walls (16) of the further compartment conveyor (14, 15), the or each further compartment conveyor (14, 15) being driven continuously in the second transport direction (19) and preferably being arranged above the intermediate conveyor (13) and/or the discharge conveyor (11).

9. The apparatus as claimed in claim 8, **characterized in that** the apparatus is configured to drive the intermediate conveyor (13) and the discharge conveyor (11) continuously and at the same speed in the second transport direction (19), and **in that** the two conveyors are configured as compartment conveyors and have dividing walls (16) for dividing compartments (17) of the respective conveyor, the apparatus being configured to drive the two conveyors in a manner which is aligned with respect to one another in such a way that the respective compartments (17) and dividing walls (16) are arranged so as to be aligned at least partially, with the result that products (10) are transported one after another by way of the feed conveyor (12) through a compartment (17) of the intermediate conveyor (13) into a compartment (17) of the discharge conveyor (11), the products (10) being braked with regard to their transport speed in the first transport direction (18) by way of partial contact with the dividing walls (16) of the intermediate conveyor (13) and the discharge conveyor (11).

10. The apparatus as claimed in claim 8 or 9, **characterized in that** the compartments (17) are dimensioned in such a way that they are only slightly wider than the respective product (10), with the result that the product (10) is supported transversely with respect to the first transport direction (18) by way of the compartment (17) or its dividing walls (16).

11. The apparatus as claimed in claim 8 or one of the other preceding claims 9 to11, **characterized in that** the compartment conveyor or the further compartment conveyors (14, 15) run obliquely with respect to the transport plane of the intermediate conveyor (13) and/or discharge conveyor (11), with the result that the dividing walls (16) of the compartment conveyor or the further compartment conveyors (14, 15) are withdrawn gradually from the products (10) during the transport of the products (10) in the second transport direction (19), in particular before the products (10) pass into a deflecting region of the further compartment conveyor (14, 15).

12. The apparatus as claimed in claim 8 or one of the other preceding claims 9 to 11, **characterized in that** a diverter (24) is arranged between the intermediate conveyor (13) and the discharge conveyor (11), for the preferably complete pushing of the products (10) onto the discharge conveyor (11) during the transport in the second transport direction (19) and/or for aligning the products (10) on the discharge conveyor (11).

13. The apparatus as claimed in claim 8 or one of the other preceding claims 9 to 12, **characterized in that** the intermediate conveyor (13) is configured in the second transport direction (19) with a shorter extent than the discharge conveyor (11), and preferably extends only in a transfer region for the products (10).

14. The apparatus as claimed in claim 9 or one of the other preceding claims 8 to 13, **characterized in that** a plurality of dividing walls (16) are combined to form compartment segments (24) with a plurality of dividing walls (16) which are spaced apart from one another.

15. The apparatus as claimed in claim 14 or one of the other preceding claims 8 to 13, **characterized in that** the compartment segments (24) are connected via a quick release fastener system to the corresponding conveyor, in particular to a toothed belt of the respective conveyor.

16. The apparatus as claimed in claim 8 or one of the other preceding claims 8 to 14, **characterized in that** in each case one additional compartment conveyor (14, 15), which is driven in the second transport direction (19), is arranged above the intermediate conveyor (13) and above the discharge conveyor (11).

## Revendications

1. Procédé de manipulation de produits (d'hygiène) (10), notamment de couches, les produits (10) étant transportés par un convoyeur d'amenée (12) dans une première direction de transport (18) dans des compartiments (17) d'un convoyeur d'évacuation (11), notamment d'un convoyeur de regroupement, qui transporte les produits (10) dans une deuxième direction de transport (19), la deuxième direction de transport (19) s'étendant transversalement, notamment perpendiculairement, à la première direction de transport (18), et au moins un convoyeur intermédiaire (13) étant agencé entre le convoyeur d'amenée (12) et le convoyeur d'évacuation (11), qui sert à réduire la vitesse de transport des produits (10) dans la première direction de transport (18), **caractérisé en ce que** des zones des produits (10) dépassant des compartiments (17) sont stabilisées par au moins un autre convoyeur à compartiments (14, 15), notamment par appui des produits (10) contre des parois de séparation (16) de l'autre convoyeur à compartiments (14, 15), le ou chaque autre convoyeur à compartiments (14, 15) étant entraîné en continu dans la deuxième direction de transport (19) et étant de préférence agencé au-dessus du convoyeur intermédiaire (13) et/ou du convoyeur d'évacuation (11).

2. Procédé selon la revendication 1, **caractérisé en ce que** le convoyeur intermédiaire (13) s'étend au moins essentiellement parallèlement à la deuxième direction de transport (19).

3. Procédé selon la revendication 1 ou 2, **caractérisé en ce que** la vitesse de transport des produits (10) dans la première direction de transport (18) est réduite par appui temporaire des produits (10) contre le convoyeur intermédiaire (13) ou des organes de celui-ci.

4. Procédé selon la revendication 1 ou l'une quelconque des autres revendications précédentes, **caractérisé en ce que** la vitesse de transport des produits (10) dans la première direction de transport (18) est réduite par frottement entre les produits (10) et le convoyeur intermédiaire (13) ou des organes de celui-ci.

5. Procédé selon la revendication 1 ou l'une quelconque des autres revendications précédentes, **caractérisé en ce que** le convoyeur intermédiaire (13) et le convoyeur d'évacuation (11) sont entraînés en continu et à la même vitesse dans la deuxième direction de transport (19), et **en ce que** les deux convoyeurs sont réalisés sous forme de convoyeurs à compartiments et présentent des parois de séparation (16) pour séparer les compartiments (17) du convoyeur respectif, les deux convoyeurs étant entraînés en étant orientés l'un par rapport à l'autre de telle sorte que les compartiments (17) et les parois de séparation (16) respectifs sont au moins partiellement agencés en alignement, de telle sorte que les produits (10) sont transportés les uns après les autres par le convoyeur d'amenée (12) à travers un compartiment (17) du convoyeur intermédiaire (13) dans un compartiment (17) du convoyeur d'évacuation (11), les produits (10) étant freinés par appui ou frottement partiel contre les parois de séparation (16) du convoyeur intermédiaire (11) et du convoyeur d'évacuation (11) en ce qui concerne leur vitesse de transport dans la première direction de transport (18).

6. Procédé selon la revendication 1 ou l'une quelconque des autres revendications précédentes, **caractérisé en ce que** le ou les autres convoyeurs à compartiments (14, 15) s'étendent en oblique par rapport au plan de transport du convoyeur intermédiaire (13) et/ou du convoyeur d'évacuation (11), de telle sorte que les parois de séparation (16) du ou des autres convoyeurs à compartiments (14, 15) sont progressivement retirées des produits (10) pendant le transport des produits (10) dans la deuxième direction de transport (19).

7. Procédé selon la revendication 1 ou l'une quelconque des autres revendications précédentes, **caractérisé en ce qu'**entre le convoyeur intermédiaire (13) et le convoyeur d'évacuation (11) est agencé un aiguillage (22) permettant de pousser les produits (10) de préférence entièrement sur le convoyeur d'évacuation (11) et/ou de les orienter sur le convoyeur d'évacuation (11) pendant le transport dans la deuxième direction de transport (19) .

8. Dispositif pour la manipulation de produits (d'hygiène) (10), notamment de couches, avec un convoyeur d'amenée (12) pour le transport des produits (10) dans une première direction de transport (18) dans des compartiments (17) d'un convoyeur d'évacuation (11), notamment d'un convoyeur de regroupement, le convoyeur d'évacuation (11) étant adapté pour transporter les produits (10) dans une deuxième direction de transport (19), et la deuxième direction de transport (19) s'étendant transversalement, notamment perpendiculairement, à la première direction de transport (18), et au moins un convoyeur intermédiaire (13) étant agencé entre le convoyeur d'amenée (12) et le convoyeur d'évacuation (11), pour réduire la vitesse de transport des produits (10) dans la première direction de transport (18), **caractérisé en ce que** des zones des produits (10) dépassant des compartiments (17) sont stabilisées par au moins un autre convoyeur à compartiments (14, 15), notamment par appui des produits (10) contre des parois de séparation (16) de l'autre convoyeur à compartiments (14, 15), le ou chaque autre convoyeur à compartiments (14, 15) étant entraîné en continu dans la deuxième direction de transport (19) et étant de préférence agencé au-dessus du convoyeur intermédiaire (13) et/ou du convoyeur d'évacuation (11).

9. Dispositif selon la revendication 8, **caractérisé en ce que** le dispositif est adapté pour entraîner le convoyeur intermédiaire (13) et le convoyeur d'évacuation (11) en continu et à la même vitesse dans la deuxième direction de transport (19), et **en ce que** les deux convoyeurs sont réalisés sous forme de convoyeurs à compartiments et présentent des parois de séparation (16) pour séparer des compartiments (17) du convoyeur respectif, le dispositif étant adapté pour entraîner les deux convoyeurs en étant orientés l'un par rapport à l'autre de telle sorte que les compartiments (17) et les parois de séparation (16) respectifs sont au moins partiellement agencés en alignement, de telle sorte que des produits (10) sont transportés les uns après les autres par le convoyeur d'amenée (12) à travers un compartiment (17) du convoyeur intermédiaire (13) dans un compartiment (17) du convoyeur d'évacuation (11), les produits (10) étant freinés par appui partiel sur les parois de séparation (16) du convoyeur intermédiaire (13) et du convoyeur d'évacuation (11) en ce qui concerne leur vitesse de transport dans la première direction de transport (18).

10. Dispositif selon la revendication 8 ou 9, **caractérisé en ce que** les compartiments (17) sont dimensionnés de telle sorte qu'ils ne sont que légèrement plus larges que le produit respectif (10), de telle sorte que le produit (10) est supporté par le compartiment (17) ou ses parois de séparation (16) transversalement à la première direction de transport (18).

11. Dispositif selon la revendication 8 ou l'une quelconque des autres revendications 9 à 11 précédentes, **caractérisé en ce que** le ou les autres convoyeurs à compartiments (14, 15) s'étendent en oblique par rapport au plan de transport du convoyeur intermédiaire (13) et/ou du convoyeur d'évacuation (11), de telle sorte que les parois de séparation (16) du ou des autres convoyeurs à compartiments (14, 15) sont progressivement retirées des produits (10) pendant le transport des produits (10) dans la deuxième direction de transport (19), notamment avant que les produits (10) n'arrivent dans une zone de déviation de l'autre convoyeur à compartiments (14, 15).

12. Dispositif selon la revendication 8 ou l'une quelconque des autres revendications 9 à 11 précédentes, **caractérisé en ce qu'**entre le convoyeur intermédiaire (13) et le convoyeur d'évacuation (11) est agencé un aiguillage (24) permettant de pousser les produits (10) de préférence entièrement sur le convoyeur d'évacuation (11) pendant le transport dans la deuxième direction de transport (19) et/ou d'orienter les produits (10) sur le convoyeur d'évacuation (11).

13. Dispositif selon la revendication 8 ou l'une quelconque des autres revendications 9 à 12 précédentes, **caractérisé en ce que** le convoyeur intermédiaire (13) est réalisé avec une extension plus courte que le convoyeur d'évacuation (11) dans la deuxième direction de transport (19) et s'étend de préférence uniquement dans une zone de transfert des produits (10).

14. Dispositif selon la revendication 9 ou l'une quelconque des autres revendications 8 à 13 précédentes, **caractérisé en ce que** plusieurs parois de séparation (16) sont regroupées en segments de compartiment (24) avec plusieurs parois de séparation (16) espacées les unes des autres.

15. Dispositif selon la revendication 14 ou l'une quelconque des autres revendications 8 à 13 précédentes, **caractérisé en ce que** les segments de compartiment (24) sont couplés au convoyeur correspondant par un système de verrouillage rapide, notamment à une courroie crantée du convoyeur respectif.

16. Dispositif selon la revendication 8 ou l'une quelconque des autres revendications 8 à 14 précédentes, **caractérisé en ce qu'**au-dessus du convoyeur intermédiaire (13) et au-dessus du convoyeur d'évacuation (11), est agencé respectivement un convoyeur à compartiments supplémentaire (14, 15), qui est entraîné dans la deuxième direction de transport (19).
